# EUROPÄISCHE PATENTSCHRIFT

(11) **EP 2 094 330 B1**
(45) Veröffentlichungstag und Bekanntmachung des Hinweises auf die Patenterteilung: **11.01.2012**
(21) Anmeldenummer: 07846660.4
(22) Anmeldetag: 19.11.2007
(51) Int. Cl.: A61M 1/16

(54) **BLUTBEHANDLUNGSGERÄT, SPEICHERMITTEL ZUR VERWENDUNG MIT DEM BLUTBEHANDLUNGSGERÄT UND KOMBINATION DES BLUTBEHANDLUNGSGERÄTS MIT DEM SPEICHERMITTEL**
BLOOD TREATMENT APPLIANCE, STORAGE MEANS FOR USING WITH THE BLOOD TREATMENT APPLIANCE, AND COMBINATION OF THE BLOOD TREATMENT APPLIANCE AND STORAGE MEANS
DISPOSITIF DE TRAITEMENT SANGUIN, ÉLÉMENT MÉMOIRE À UTILISER AVEC LE DISPOSITIF DE TRAITEMENT SANGUIN ET COMBINAISON DU DISPOSITIF DE TRAITEMENT SANGUIN ET DE L'ÉLÉMENT MÉMOIRE

(30) Priorität: 20.11.2006 DE 102006054872
(43) Veröffentlichungstag der Anmeldung: 02.09.2009
(73) Patentinhaber: Fresenius Medical Care Deutschland GmbH, 61352 Bad Homburg (DE)
(72) Erfinder: BROWN, Gail-Suzanne, 61250 Usingen-Eschbach (DE); SPICKERMANN, Reiner, 97535 Wasserlosen-Burghausen (DE)
(74) Vertreter: Ziermann, Oliver
(86) Internationale Anmeldenummer: PCT/EP2007/009981
(87) Internationale Veröffentlichungsnummer: WO 2008/061685

(56) Entgegenhaltungen:
- EP-A- 0 857 490
- EP-A- 1 195 708
- EP-A- 1 473 664
- DE-A1-102004 011 264
- US-A1- 2005 020 961
- US-A1- 2006 023 433

## Beschreibung

Die Erfindung betrifft das Gebiet von Blutbehandlungsgeräten nach dem Oberbegriff des Anspruchs 1.

Bei derartigen Blutbehandlungsgeräten wird Blut von Patienten zum Zweck der Blutbehandlung über einen extrakorporalen Blutkreislauf zunächst einem Patienten entzogen, an einer Blutbehandlungseinheit einer Behandlung entzogen und dann wieder dem Patienten zugeführt. Ein Beispiel hierfür ist die in der Nierenersatzbehandlung eingesetzte Hämodialyse. Bei der Hämodialyse ist die Blutbehandlungseinheit ein Hämodialysator, der durch eine semipermeable Membran in eine vom Blut des Patienten durchflossene Blutkammer und eine von einer Reinigungsflüssigkeit, der Dialysierflüssigkeit, durchflossene Dialysierflüssigkeitskammer unterteilt ist. Die semipermeable Membran besteht dabei meist aus Tausenden einzelner Hohlfasern. Die von dem Blut zu entfernenden Substanzen treten durch Diffusion in die Dialysierflüssigkeit über und werden mit dieser abgeführt. Gleichzeitig kann durch einen Druckgradienten zu entfernende Flüssigkeit dem Blut entzogen werden. Das in diesem Fall als Hämodialysegerät ausgebildete Blutbehandlungsgerät steuert die Bereitstellung der Dialysierflüssigkeit in der richtigen Zusammensetzung und Temperatur sowie den Flüssigkeitsentzug. Gleichzeitig wird der Blutfluss im extrakorporalen Blutkreislauf durch Ansteuerung einer meist als Rollenpumpe ausgeführten Blutpumpe kontrolliert sowie auf Alarmzustände überwacht. Abwandlungen in dieser Form der Nierenersatzbehandlung betreffen die Hämofiltration oder Hämodiafiltration, bei denen anstelle oder zusätzlich zum diffusen Reinigungsprinzip ein Flüssigkeitsaustausch durch erhöhten Flüssigkeitsentzug und gleichzeitigen Austausch mit einer Substitutionslösung durchgeführt wird. Auch die hierbei eingesetzte Blutbehandlungseinheit soll im Folgenden generell als Hämodialysator bezeichnet werden.

Neben den genannten Blutbehandlungsverfahren sind weitere Blutbehandlungsverfahren bekannt, bei denen Blut in einem extrakorporalen Kreislauf einer Behandlung unterzogen wird. Als Beispiele sind der Einsatz von Oxygenatoren zur künstlichen Beatmung, von Adsorbersäulen zur Blutreinigung oder von Blutzentrifugen zum Abtrennen von Blutbestandteilen nicht nur zur therapeutischen, sondern auch zu Spenderzwecken zu nennen.

Allen diesen Behandlungsformen ist gemeinsam, dass der Patient, wobei im Folgenden auch Spender darunter verstanden werden, längere Zeit mit dem Blutbehandlungsgerät zur Durchführung der Blutbehandlung verbunden ist. Die entsprechende Zeitspanne kann bei mehreren Stunden liegen, in der chronischen Nierenersatzbehandlung liegt sie regelmäßig bei ca. vier Stunden. Bei solchen Behandlungen ist es weit verbreitet, sie in Kliniken durchzuführen, die die Patienten zur ambulanten Behandlung regelmäßig - durchschnittlich dreimal pro Woche - aufsuchen. Dabei werden oft mehrere Patienten gleichzeitig in einem größeren Raum behandelt, wobei sich die Patienten aufgrund der permanenten Verbindung mit dem Blutbehandlungsgerät vom Behandlungsplatz nicht entfernen können.

Die Patienten verbringen die Behandlungszeit im Allgemeinen durch Fernsehen, Lesen oder Schlafen.

Um eine einfache Bedienbarkeit der Dialysemaschine oder zumindest die Erfassung des Gerätezustandes zu ermöglichen, schlägt die US 5,903,211 vor, ein externes Unterhaltungsgerät wie zum Beispiel ein Fernsehgerät so mit einem Hämodialysegerät zu verbinden, dass auf dem Fernsehgerät anstelle des Unterhaltungsprogramms die Einstellungen des Hämodialysegerätes abrufbar und sogar änderbar sind.

Ein persönliches Einrichten der direkten Umgebung eines Behandlungsplatzes ist aufgrund der hygienischen Randbedingungen sowie des ständigen Wechsels der Patienten in einer Klinik aber praktisch nicht möglich. Insbesondere ist eine permanente Einrichtung mit individuellen Gegenständen wie Bildern aus diesem Grund nicht realisierbar, und ein ständiges Mitbringen und wieder Mitnehmen derartiger Gegenstände ist viel zu aufwändig. EP-A-1 195 708 beschreibt ein Blutbehandlungssystem, bei dem das Benutzer-Interface zur Benutzung durch das Klinikpersonal über einen Web-Browser auf einen Webserver in dem Blutbehandlungssystem zugreift. Der Webserver macht so Informationen über das Blutbehandlungssystem dem Klinikpersonal zugänglich. Über das Benutzer-Interface kann Klinikpersonal das Blutbehandlungssystem bedienen, etwa zur Veränderung der Infusionsrate. Ein Zugriff auf den Webserver kann darüber hinaus von einer entfernten Stelle aus über einen externen Web Browser erfolgen. Auf diese Weise wird die Datenübertragung vereinheitlicht.

Der externe Browser ermöglicht beispielsweise den Zugriff eines Service-Technikers, der so eine Behandlung verfolgen, technische Unterstützung geben oder Wartungsoperationen initiieren kann. Um einen unbeabsichtigten Eingriff eines Servicetechnikers in den Behandlungsablauf zu verhindern, werden in dem Web Server unterschiedliche Autorisierungen gesetzt, die auf der Herkunft des Befehls beruhen.

Zusätzlich zu dem Benutzer-Interface für das Klinikpersonal kann ein zweites Benutzer-Interface für die Benutzung von angeschlossenen Datennetzen durch den Patienten vorgesehen sein.

Der Erfindung liegt die Aufgabe zu Grunde, ein gattungsgemäßes Blutbehandlungsgerät derart weiterzubilden, dass eine gewisse Individualisierung des Behandlungsplatzes ohne oder nur mit geringem Mehraufwand ermöglicht wird, um das Behandlungsumfeld für einen mit dem Gerät zu behandelnden Patienten angenehmer zu gestalten.

Nach der Lehre der Erfindung wird diese Aufgabe durch ein Blutbehandlungsgerät mit den Merkmalen des Anspruchs 1, eine Verwendung eines externen Speichermittels mit dem erfindungsgemäßen Blutbehandlungsgerät mit den Merkmalen des Anspruchs 12 sowie eine Kombination eines Blutbehandlungsgeräts mit einem externen Speichermittel mit den Merkmalen des Anspruchs 14 gelöst. Vorteilhafte Ausgestaltungen sind Gegenstand der Unteransprüche.

Der Erfindung liegt die Beobachtung zu Grunde, dass zur Individualisierung eines Behandlungsplatzes ein entscheidender Beitrag dadurch geleistet werden kann, wenn es dem Patienten ermöglicht wird, während der Behandlung von ihm ausgewählte Bilder oder Ansichten betrachten zu können. Da die heutigen Blutbehandlungsgeräte im Allgemeinen mit ausreichendem Speicherplatz für Steuerprogramme und Daten sowie einer als Bildschirm ausgestalteten Anzeigeeinheit ausgerüstet sind, können entsprechende Bilddateien auf dem Bildschirm des Blutbehandlungsgeräts eingezeigt werden. Die Bilddateien können von dem Patienten auf einer meist sowieso vorhandenen Chipkarte zur Speicherung von Patienten- und/oder Behandlungsdaten oder auf anderen geeigneten Speichermedien wie zum Beispiel einem USB-Stick mitgebracht werden. Dabei kann es auch ausreichend sein, dass die Bilddateien bereits auf einem anderen Speichermittel gespeichert sind, das mit der Steuereinheit des Blutbehandlungsgerätes verbunden ist, und sich die vom Patienten mitgebrachten Daten auf eine indizierte Auswahl der anzuzeigenden Bilder beschränken. Die Auswahl oder die Bilddateien werden über eine mit der Steuereinheit verbundene Datenschnittstelle in das Blutbehandlungsgerät übertragen oder von der Steuereinheit bei Bedarf abgefragt. Die Steuereinheit ist erfindungsgemäß so konfiguriert, dass sie die so ausgewählten Bilddateien während der Blutbehandlung auf dem Bildschirm des Blutbehandlungsgerätes anzeigt. Dies kann in einer fest vorgegebenen Sequenz im Sinne einer Diashow geschehen oder durch eine individuelle Vorgabe des Patienten vorbestimmt sein. Im einfachsten Fall kann auch nur die Darstellung eines einzigen Bildes erfolgen. Nach Beendigung der Behandlung kann die Darstellung der Bilddateien automatisch beendet werden, so dass keinerlei Umrüstung erforderlich ist. Vielmehr wird durch die Initiierung der nächsten Behandlung und durch die Übergabe der individuellen Bilddateien oder zumindest der individuellen Bildindizierung eine Anpassung der Umgebung auch beim nachfolgenden Patienten auf einfache Art und Weise erreicht. Aufgrund der erfindungsgemäßen Gestaltung des Blutbehandlungsgerätes gelingt damit eine Individualisierung des Behandlungsplatzes ohne entscheidenden Mehraufwand.

In einer Ausführungsform der Erfindung weist das Blutbehandlungsgerät interne Speichermittel zum Speichern der Bilddateien auf, die mit der Steuereinheit zum Abruf der Bilddateien verbunden ist.

In einer weiteren Ausführungsform ist die Datenschnittstelle eine USB-Schnittstelle. Dieser weit verbreitete Schnittstellentyp ermöglicht die Anbindung insbesondere von Datenträgern wie einem USB-Stick. Ein solcher USB-Stick ist ein Beispiel für den Anschluss der Datenschnittstelle an ein externes Speichermittel. Hier kann auch jedes andere Speichermittel in Betracht kommen. Diese können flexibel und mobil einsetzbar wie ein USB-Stick sein, wie zum Beispiel in Form einer Speicherchipkarte. Es ist auch denkbar, eine Datenübertragung zu einem entfernten Speicher über ein Netzwerk zu nutzen.

Die Verwendung einer Speicherchipkarte hat den Vorteil, dass eine solche Karte bei den auf dem Markt befindlichen Blutbehandlungsgeräten verbreitet als so genannte Patientenkarte zur Speicherung von Behandlungs- und/oder Patientendaten bereits eingesetzt wird. Diese Karte kann in einer besonders vorteilhaften Ausführungsform der Erfindung auch als externes Speichermittel zum Übertragen oder der Indizierung der anzuzeigenden Bilddateien verwendet werden.

Die Steuereinheit kann ferner so ausgelegt sein, dass sie die Bilddateien als Hintergrundbild auf dem Bildschirm zur Anzeige bringt. Dabei können zur Darstellung der Bilddateien angezeigte Blutbehandlungsinformationen teilweise oder vollständig ausgeblendet werden. In diesem Fall kann die Steuereinheit so ausgelegt sein, dass sie beim Eintritt von vordefinierten Bedingungen die Ausblendung rückgängig macht und/oder neue Behandlungsinformationen anzeigt. Derartige vordefinierte Bedingungen können Alarmzuständen entsprechen, und die neuen Behandlungsinformationen können Alarmsignale darstellen. Auch ist es vorteilhaft, wenn die vordefinierten Bedingungen die Betätigung der Eingabemittel umfassen. Auf diese Weise ergibt sich für den Benutzer sofort das normale Erscheinungsbild auf dem Bildschirm, wenn er die Eingabemittel betätigt. Die Eingabemittel können dabei als vom Bildschirm separate Elemente wie zum Beispiel Tasten oder Drehknöpfe ausgeführt sein. Die Eingabemittel können aber auch den Bildschirm selbst umfassen, wenn dieser als berührungsempfindlicher Bildschirm (Touchscreen) ausgeführt ist.

Die Erfindung umfast gleichermaßen die verwendung eines externen Speichermittels die verwendung eines mit dem erfindungsgemäßen Blutbehandlungsgerät, das zum Anschluss an die Datenschnittstelle des Blutbehandlungsgerätes zur Übertragung und/oder Indizierung der auf dem Bildschirm während einer Blutbehandlung darzustellenden Bilddateien ausgebildet ist. Dieses externe Speichermittel ist dabei bevorzugt - wie bereits erläutert - als USB-Stick oder als eine Speicherchipkarte ausgeführt.

Weiterhin umfasst die Erfindung eine Kombination bestehend aus dem erfindungsgemäßen Blutbehandlungsgerät und einem externen Speichermittel.

Weitere Einzelheiten und Vorteile der Erfindung werden anhand eines in den Zeichnungen dargestellten Ausführungsbeispiels näher beschrieben. Es zeigen:
Fig. 1 eine schematische Darstellung einer Ausführungsform des erfindungsgemäßen Blutbehandlungsgerätes;
Fig. 2a eine erste Ansicht des Bildschirms der Ausführungsform des erfindungsgemäßen Blutbehandlungsgerätes, bei dem Behandlungsinformationen angezeigt werden; und
Fig. 2b eine zweite Ansicht des Bildschirms der Ausführungsform des erfindungsgemäßen Blutbehandlungsgerätes, bei dem eine Bilddatei angezeigt wird.

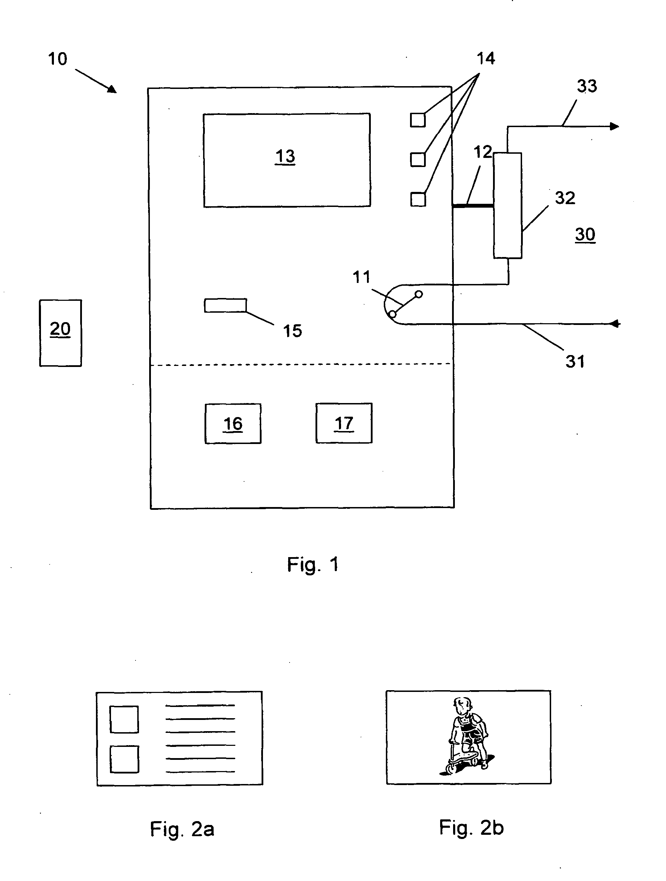

In Fig. 1 ist eine Ausführungsform eines erfindungsgemäßen Blutbehandlungsgerätes 10 zu sehen. Das Blutbehandlungsgerät 10 dient zur Durchführung einer Blutbehandlung mit Hilfe eines extrakorporalen Blutkreislaufs 30, der nicht direkt Bestandteil des Blutbehandlungsgerätes 10 ist, sondern dessen Komponenten nach jeder Behandlung verworfen werden. Der extrakorporale Blutkreislauf umfasst eine Blutzuführleitung 31, um das Blut eines Patienten einer Blutbehandlungseinheit 32 zuzuführen, sowie eine Blutabführleitung 33 zur Rückführung des Blutes. Das Blutbehandlungsgerät 10 weist Mittel zum Fördern des Blutes in dem extrakorporalen Blutkreislauf in Form einer Blutpumpe 11 auf. In Fig. 1 sind dabei nur die Komponenten dargestellt, die zum Verständnis der Erfindung erforderlich sind. Dem Fachmann ist geläufig, dass jeweils weitere Komponenten hinzutreten, um die unterschiedlichsten Aufgaben bei einer Blutbehandlung wahrzunehmen.

Das Blutbehandlungselement 32 ist im Fall, dass das Blutbehandlungsgerät 10 ein Hämodialysegerät ist, ein Hämodialysator. In Fig. 1 ist schematisch ein Halter 12 zur Halterung des Blutbehandlungselementes 32 an dem Blutbehandlungsgerät 10 gezeigt. Eventuell weitere Anschlüsse wie im Fall der Hämodialyse für die Zu- und Abführung der Dialysierflüssigkeit sind unterdrückt.

Das Blutbehandlungsgerät 10 umfasst einen Bildschirm 13, der als berührungsempfindlicher Bildschirm ausgebildet ist, um gleichzeitig als Eingabemittel zu dienen. Es sind ferner noch einige als so genannte Hardkeys 14, zum Beispiel in Form von Tasten, ausgebildete Eingabemittel neben dem Bildschirm 13 vorgesehen. Auf der Frontseite des Blutbehandlungsgerätes 10 ist ferner eine Datenschnittstelle 15 vorhanden, die als Chipkartenlesegerät oder USB-Schnittstelle ausgebildet ist. Schematisch angedeutet sind ferner einer Steuereinheit 16 und ein internes Speichermittel 17, die sich im Inneren des Blutbehandlungsgerätes 10 befinden. Die Steuereinheit 16, das interne Speichermittel 17, der Bildschirm 13 und die Datenschnittstelle 15 sind über einen nicht gezeigten Kommunikationsbus miteinander verbunden.

Das erfindungsgemäße Blutbehandlungsgerät 10 arbeitet nun wie folgt: Zu einer Blutbehandlung, zum Beispiel einer Hämodialysebehandlung, bringt der Patient ein als Speicherchipkarte 20 ausgebildetes externes Speichermittel mit. Auf der Speicherchipkarte 20 können Patientendaten wie Name, Größe, Gewicht sowie Daten über die vergangenen Behandlungen wie Zeitpunkt, Dauer und Behandlungsdosis als auch die vom Arzt verschriebenen Daten über die durchzuführende Behandlung gespeichert sein. Auf diese Daten hat der Patient im Allgemeinen keinen direkten Zugriff, zumindest kann er sie nur eingeschränkt oder gar nicht ohne besondere Autorisierung ändern. Neben diesen Daten sind auf der Speicherchipkarte 20 aber auch Bilddateien abgespeichert, die der Patient in einem für diesen Zweck reservierten Speicherbereich zuvor auf die Speicherchipkarte 20 geladen hat.

Die Speicherchipkarte wir nun vor der Behandlung in die Datenschnittstelle 15 eingeführt, die als Chipkartenlese- und -schreibgerät ausgebildet ist. Die Steuereinheit 16 ist so konfiguriert, dass sie die Behandlungsdaten für die durchzuführende Behandlung von der Speicherchipkarte 20 einlesen und in dem internen Speichermittel 17 abspeichern kann. Die Bilddateien, die vorzugsweise in einem geläufigen und komprimierten Format wie zum Beispiel JPEG, TIFF oder PDF auf der Chipkarte abgespeichert sind, werden ebenfalls auf die internen Speichermittel 17 übertragen. Zusätzlich können Informationen auf der Speicherchipkarte 20 hinterlegt sein, in welcher Reihenfolge die Bilddateien später auf dem Bildschirm 13 dargestellt werden sollen. Es kann auch vorgesehen sein, dass dem Patienten auf dem Bildschirm 13 eine Art Auswahlverzeichnis der zur Verfügung stehenden Bilddateien angezeigt wird und dieser die anzuzeigenden Sequenzen mit Hilfe des berührungsempfindlichen Bildschirms 13 und/oder der Hardkeys 14 auswählen kann. Dem Fachmann stehen hierfür unterschiedlichste Auswahltechniken zur Verfügung. In dem internen Speichermittel 17 kann dabei ein Speicherbereich für jeden Patienten reserviert sein, so dass die Bilddateien auch für nachfolgende Behandlungen zur Verfügung stehen können. Dann kann es ausreichend sein, lediglich die Bildsequenzen auf der Speicherchipkarte 20 indiziert abzuspeichern, um bereits in dem internen Speichermittel 17 abgespeicherte Bilddateien während einer Behandlung anzeigen zu können.

Bei der Ausführungsform der Datenschnittstelle 15 als USB-Schnittstelle bietet es sich an, die Bilder auf dem externen Speichermittel, das in diesem Fall in Form eines USB-Sticks mit immenser Speicherkapazität vorliegen kann, abgespeichert zu lassen. Im Prinzip können die Bilddateien bei ausreichender Speicherkapazität auch als Filmdatei vorliegen, wobei im Rahmen dieser Patentanmeldung die Bezeichnung "Bilddatei" explizit auch Filmdateien umfassen soll.

Nach dem Anschluss des Patienten an die Leitungen des extrakoporalen Blutkreislaufs 30 sowie des Beginns der Blutbehandlung beginnt die Steuereinheit 16 damit, die zuvor verwendete Bildschirmansicht zur Anzeige der Behandlungsinformationen (Behandlungsinformationsmodus), wie sie äußerst schematisch in Fig. 2a dargestellt ist, auszublenden. Stattdessen erfolgt die Einblendung der vom Patienten ausgewählten und eingespielten Bilddateien (Bilddarstellungsmodus), wie dies schematisch in Fig. 2b gezeigt ist. Dabei können auch einige wenige, besonders wichtige Behandlungsdaten eingeblendet bleiben (nicht gezeigt), so dass sich die Bilder als Hintergrundbild zeigen.

Sollte die Steuereinheit 16 feststellen, dass aufgrund des Eintretens von vorgegebenen Bedingungen wie zum Beispiel Alarmzuständen, das Berühren des Bildschirms 13 oder das Betätigen der Hardkeys 14 zur Anzeige von Behandlungsinformationen zurückgekehrt werden soll, so ändert sie die Bildschirmdarstellung entsprechend. Insbesondere im Fall von Alarmzuständen werden gegebenenfalls gleichzeitig weitere optische und/oder akustische Signale abgegeben, um die Aufmerksamkeit des Patienten oder des Bedienpersonals auf den Alarmzustand zu lenken.

Durch Betätigung eines bestimmten Eingabemittels wie zum Beispiel eines bestimmten Hardkeys 14 kann der Anwender die Rückkehr in den Bilddarstellungsmodus herbeiführen. Unter bestimmten Umständen kann die Steuereinheit 16 auch nach Ablauf einer vorgegebenen Zeitspanne selbstständig in diesen Anzeigemodus wechseln. Auch kann vorgesehen sein, dass der Bilddarstellungsmodus von der Steuereinheit 16 generell als eine Art Bildschirmschoner nach einer vorgegebenen Zeitspanne aktiviert wird.

Auch kann vorgesehen sein, dass die Steuereinheit 16 bei Unterbrechung der Datenschnittstelle 15 zum Beispiel nach dem Entfernen des externen Speichermittels 20 sofort in den Behandlungsinformationsmodus wechselt und gegebenenfalls einen Zugang zu den für den Patienten in dem internen Speichermittel 17 gespeicherten Bilddateien sperrt.

Mit Hilfe des erfindungsgemäßen Blutbehandlungsgerätes gelingt es, den Behandlungsplatz für einen Patienten, der sich ambulant einer mehrere Stunden andauernden Blutbehandlung unterziehen muss, individualisiert zu gestalten und somit für ein gesteigertes Wohlfühlempfinden beim Patienten während der Blutbehandlungen zu sorgen. Durch die Wiedergabe von dem Patienten geläufigen Bildern zum Beispiel durch die Darstellung von Verwandten oder Landschaftsbildern ist eine Aufwertung des ansonsten eher wenig individuell wirkenden Behandlungsumfeldes möglich.

## Patentansprüche

1. Blutbehandlungsgerät (10) zur Blutbehandlung eines Patienten, bei der Blut von einem Patienten zu einer Blutbehandlungseinheit (32) und von dieser dem Patienten wieder zugeführt wird, mit
einem Bildschirm (13) zur Anzeige von die Blutbehandlung betreffenden Informationen
Eingabemitteln (13, 14),
einer elektronischen Datenschnittstelle (15) und
einer mit der elektronischen Datenschnittstelle (15) und dem Bildschirm (13) verbundenen Steuereinheit (16),
**dadurch gekennzeichnet,**
**dass** die Steuereinheit (16) so konfiguriert ist, in einem Behandlungsinformationsmodus Behandlungsinformationen auf dem Bildschirm anzuzeigen und in einem Bilddarstellungsmodus über die Datenschnittstelle (15) übertragene und/oder indizierte und von dem Patienten ausgewählte und eingespielte Bilddateien auf dem Bildschirm (13) während einer Blutbehandlung darzustellen.

2. Blutbehandlungsgerät nach Anspruch 1, **dadurch gekennzeichnet, dass** die Blutbehandlungseinheit (32) ein Hämodialysator ist.

3. Blutbehandlungsgerät nach einem Ansprüche 1 oder 2, **dadurch gekennzeichnet, dass** es ein mit der Steuereinheit (16) verbundenes internes Speichermittel (17) zum Speichern der Bilddateien aufweist.

4. Blutbehandlungsgerät nach einem der vorhergehenden Ansprüche, **dadurch gekennzeichnet, dass** die Datenschnittstelle (15) zum Anschluss an ein vom Blutbehandlungsgerät externes Speichermittel (20) ausgelegt ist.

5. Blutbehandlungsgerät nach Anspruch 4, **dadurch gekennzeichnet, dass** die Datenschnittstelle (15) eine USB-Schnittstelle ist.

6. Blutbehandlungsgerät nach einem der Ansprüche 4 oder 5, **dadurch gekennzeichnet, dass** das externe Speichermittel (20) eine Speicherchipkarte oder ein USB-Speicherstick ist.

7. Blutbehandlungsgerät nach einem der vorhergehenden Ansprüche, **dadurch gekennzeichnet, dass** die Steuereinheit (16) so ausgelegt ist, dass sie die von dem Patienten ausgewählten und eingespielten Bilddateien als Hintergrundbild auf dem Bildschirm (13) zur Anzeige bringt.

8. Blutbehandlungsgerät nach einem der vorhergehenden Ansprüche, **dadurch gekennzeichnet, dass** die Steuereinheit (16) so ausgelegt ist, dass sie zur Darstellung der von dem Patienten ausgewählten und eingespielten Bilddateien angezeigte Blutbehandlungsinformationen zum Teil oder vollständig ausblendet.

9. Blutbehandlungsgerät nach Anspruch 8, **dadurch gekennzeichnet, dass** die Steuereinheit (16) ferner so ausgelegt ist, dass sie beim Eintritt von vordefinierten Bedingungen die Ausblendung rückgängig macht und/oder neue Behandlungsinformationen anzeigt.

10. Blutbehandlungsgerät nach Anspruch 9, **dadurch gekennzeichnet, dass** die vordefinierten Bedingungen Alarmzuständen entsprechen und die neuen Behandlungsinformationen Alarmsignale darstellen.

11. Blutbehandlungsgerät nach Anspruch 8 oder 9, **dadurch gekennzeichnet, dass** die vordefinierten Bedingungen die Betätigung der Eingabemittel (13, 14) umfassen.

12. Verwendung eines externen Speichermittels (20) mit einem Blutbehandlungsgerät (10) nach einem der Ansprüche 4 - 6, enthaltend die folgenden Schritte: Anschließen des externen Speichermittels an die Datenschnittstelle (15) des Blutbehandlungsgerätes (10) und Übertragung und/oder Indizierung der auf dem Bildschirm (13) des Blutbehandlungsgerätes (10) während einer Blutbehandlung darzustellenden Bilddateien.

13. Verfahren nach Anspruch 12, **dadurch gekennzeichnet, dass** das externe Speichermittel ein USB-Stick oder eine Speicherchipkarte ist.

14. Kombination aus einem Blutbehandlungsgerät nach einem der Ansprüche 4 bis 6 und einem externen Speichermittels (20) zur Verwendung mit dem Blutbehandlungsgerät (10), wobei das externe Speichermittel zum an die Datenschnittstelle (15) des Blutbehandlungsgerätes (10) und zur Übertragung und/oder Indizierung der auf dem Bildschirm (13) des Blutbehandlungsgerätes (10) während einer Blutbehandlung darzustellenden Bilddateien ausgebildet ist.

## Claims

1. A blood treatment device (10) for treating the blood of a patient, wherein blood from a patient is supplied to a blood treatment unit (32) and is sent back to the patient from the latter, comprising
a display screen (13) for displaying information pertaining to the blood treatment,
input means (13, 14),
an electronic data interface (15) and
a control unit (16), which is connected to the electronic data interface (15) and to the display screen (13),
**characterized in that**
the control unit (16) is configured so that in a treatment information mode, treatment information is to be displayed on the display screen, and in an image display mode, image files that have been transmitted over the data interface (15) and/or have been indexed and selected by the patient and loaded are to be displayed on the display screen (13) during a blood treatment.

2. The blood treatment device according to Claim 1, **characterized in that** the blood treatment unit (32) is a hemodialyzer.

3. The blood treatment device according to any one of Claims 1 or 2, **characterized in that** it has an internal memory means (17) which is connected to the control unit (16) for storing the image data files.

4. The blood treatment device according to any one of the preceding claims, **characterized in that** the data interface (15) is designed for being connected to a memory means (20) that is external to the blood treatment device.

5. The blood treatment device according to Claim 4, **characterized in that** the data interface (15) is a USB interface.

6. The blood treatment device according to any one of Claims 4 or 5, **characterized in that** the external memory means (20) is a memory chip card or a USB memory stick.

7. The blood treatment device according to any one of the preceding claims, **characterized in that** the control unit (16) is designed so that it displays the image data files selected by the patient and shown as a background image on the display screen (13) .

8. The blood treatment device according to any one of the preceding claims, **characterized in that** the control unit (16) is designed so that it partially or completely masks out blood treatment information displayed for representing the image data files selected by the patient.

9. The blood treatment device according to Claim 8, **characterized in that** the control unit (16) is also designed so that it reverses the masking on occurrence of predefined conditions and/or displays new treatment information.

10. The blood treatment device according to Claim 9, **characterized in that** the predefined conditions correspond to alarm states, and the new treatment information represents alarm signals.

11. The blood treatment device according to Claim 8 or 9, **characterized in that** the predefined conditions comprise the operation of the input means (13, 14).

12. Use of an external memory means (20) with a blood treatment device (10) according to any one of Claims 4 to 6, comprising the following steps: connecting the external memory means to the data interface (15) of the blood treatment device (10) and transferring and/or indexing the image data files to be represented on the display screen (13) of the blood treatment device (10) during a blood treatment.

13. A method according to Claim 12, **characterized in that** the external memory means is a USB stick or a memory chip card.

14. A combination of a blood treatment device according to any one of Claims 4 to 6 and an external memory means (20) for use with the blood treatment device (10), wherein the external memory means is designed for connection to the data interface (15) of the blood treatment device (10) and for transmitting and/or indexing the image data files to be represented on the display screen (13) of the blood treatment device (10) during a blood treatment.

## Revendications

1. Appareil de traitement sanguin (10) pour le traitement sanguin d'un patient, dans lequel le sang d'un patient est acheminé vers une unité de traitement sanguin (32) et renvoyé de cette dernière vers le patient, comportant
un écran (13) pour l'affichage d'informations concernant le traitement sanguin,
des moyens de saisie (13, 14),
une interface électronique de données (15) et
une unité de commande (16) reliée à l'interface électronique de données (15) et à l'écran (13),
**caractérisé en ce que**
l'unité de commande (16) est configurée de manière à afficher des informations de traitement à l'écran en mode d'informations de traitement et à représenter à l'écran (13) en mode de représentation d'image pendant un traitement sanguin des données d'image transférées via l'interface de données (15) et/ou indiquées et sélectionnées par le patient et rentrées.

2. Appareil de traitement sanguin selon la revendication 1, **caractérisé en ce que** l'unité de traitement sanguin (32) est un hémodyaliseur.

3. Appareil de traitement sanguin selon une des revendications 1 ou 2, **caractérisé en ce qu'**il présente un moyen de sauvegarde interne (17) relié à l'unité de commande (16) pour sauvegarder les données graphiques.

4. Appareil de traitement sanguin selon une des revendications précédentes, **caractérisé en ce que** l'interface de données (15) est conçue pour être raccordée à un moyen de sauvegarde (20) extérieur à l'appareil de traitement sanguin.

5. Appareil de traitement sanguin selon la revendication 4, **caractérisé en ce que** l'interface de données (15) est une interface USB.

6. Appareil de traitement sanguin selon une des revendications 4 ou 5, **caractérisé en ce que** le moyen de sauvegarde externe (20) est une carte à puces de sauvegarde ou une clé USB de sauvegarde.

7. Appareil de traitement sanguin selon une des revendications précédentes, **caractérisé en ce que** l'unité de commande (16) est conçue de manière à faire afficher à l'écran (13) en image d'arrière-plan les données d'images sélectionnées par le patient et rentrées.

8. Appareil de traitement sanguin selon une des revendications précédentes, **caractérisé en ce que** l'unité de commande (16) est conçue de manière à masquer partiellement ou totalement les informations de traitement sanguin pour l'affichage des données d'images sélectionnées par le patient et rentrées.

9. Appareil de traitement sanguin selon la revendication 8, **caractérisé en ce que** l'unité de commande (16) est par ailleurs conçue de manière à annuler le masquage rétroactif et/ou à afficher de nouvelles informations de traitement en cas de survenance de conditions prédéfinies.

10. Appareil de traitement sanguin selon la revendication 9, **caractérisé en ce que** les conditions prédéfinies correspondent à des états d'alarme et les nouvelles informations de traitement constituent des signaux d'alarme.

11. Appareil de traitement sanguin selon la revendication 8 ou 9, **caractérisé en ce que** les conditions prédéfinies recouvrent l'actionnement des moyens de saisie (13, 14).

12. Utilisation d'un moyen de sauvegarde externe (20) avec un appareil de traitement sanguin (10) selon une des revendications 4 à 6, comprenant les étapes suivantes : raccordement du moyen de sauvegarde externe à l'interface de données (15) de l'appareil de traitement sanguin (10) et transfert et/ou présentation des données d'image à représenter à l'écran (13) de l'appareil de traitement sanguin (10) pendant un traitement sanguin.

13. Procédé selon la revendication 12, **caractérisé en ce que** le moyen de sauvegarde externe est une clé USB ou une carte à puce de sauvegarde.

14. Combinaison d'un appareil de traitement sanguin selon une des revendications 4 à 6 et d'un moyen de sauvegarde externe (20) s'utilisant avec l'appareil de traitement sanguin (10), le moyen de sauvegarde externe étant conçu pour être raccordé à l'interface de données (15) de l'appareil de traitement sanguin (10) et pour le transfert et/ou la présentation des données d'image à représenter à l'écran (13) de l'appareil de traitement sanguin (10) pendant un traitement sanguin.
